# EUROPEAN PATENT APPLICATION

(11) **EP 4 289 810 A1**
(43) Date of publication of application: **13.12.2023**
(21) Application number: 22749821.9
(22) Date of filing: 04.02.2022
(51) Int. Cl.: C07C 41/30, C07C 41/42, C07C 43/16, C07B 61/00

(54) **METHOD FOR PRODUCING (2-METHOXYETHYL) VINYL ETHER, AND METHOD FOR REFINING (2-METHOXYETHYL) VINYL ETHER**

(30) Priority: 04.02.2021 JP 2021016616
(71) Applicant: Maruzen Petrochemical Co., Ltd., Tokyo 104-8502 (JP)
(72) Inventor: TENJIMBAYASHI, Ryuichi, Ichihara-shi, Chiba 290-8503 (JP); SAITO, Kyohei, Ichihara-shi, Chiba 290-8503 (JP); ITO, Yudai, Ichihara-shi, Chiba 290-8503 (JP); NANIKI, Takashi, Ichihara-shi, Chiba 290-8503 (JP); SATO, Tomohiko, Ichihara-shi, Chiba 290-8503 (JP)
(74) Representative: Wächtershäuser & Hartz Patentanwaltspartnerschaft mbB
(86) International application number: PCT/JP2022/004490
(87) International publication number: WO 2022/168950

(57) **Abstract**

Provided is a method for producing (2-methoxyethyl) vinyl ether with a high purity from 2-methoxyethanol and acetylene.

The present invention is a method for producing (2-methoxyethyl) vinyl ether using 2-methoxyethanol as a raw material alcohol, the method including the following steps A1, A2, and A3:
(Step A1) a vinyl ether synthesis step of causing 2-methoxyethanol to react with acetylene to obtain a mixture containing unreacted 2-methoxyethanol and (2-methoxyethyl) vinyl ether;
(Step A2) an acetalization step of causing a reaction of the unreacted 2-methoxyethanol and the (2-methoxyethyl) vinyl ether in the mixture in the presence of an acid catalyst to be converted into acetaldehyde bis(2-methoxyethyl)acetal;
(Step A3) a distillation step of removing the acetaldehyde bis(2-methoxyethyl)acetal by distillation from an acetal-containing mixture obtained in step A2.

## Description

### Technical Field

The present invention relates to a method for producing (2-methoxyethyl) vinyl ether and a method for purifying (2-methoxyethyl) vinyl ether.

### Background Art

As a method for producing vinyl ether, an addition reaction of an alcohol to acetylene is widely known. This method makes it difficult to purify the vinyl ether by distillation when a raw material alcohol remaining in a resulting crude vinyl ether forms an azeotropic mixture with a target vinyl ether.

As an alcohol forming the azeotropic mixture and vinyl ether corresponding to the alcohol, for example, a combination of 2-ethylhexanol and 2-ethylhexyl vinyl ether and a combination of cyclohexanol and cyclohexyl vinyl ether are known. Accordingly, a method has been proposed that causes unreacted 2-ethylhexanol or cyclohexanol contained in a mixture obtained by an addition reaction of 2-ethylhexanol or cyclohexanol to acetylene to react with 2-ethylhexyl vinyl ether or cyclohexyl vinyl ether produced by the addition reaction described above in the presence of an acid catalyst to be converted into acetal and then remove the acetal (Patent Literature 1).

### Citation List

### Patent Literature

Patent Literature 1: JP 5312133 B2

### Summary of the Invention

### Problem to be Solved by the Invention

However, there are few reports on a production of (2-methoxyethyl) vinyl ether from 2-methoxyethanol and acetylene.

The present invention provides a method for producing (2-methoxyethyl) vinyl ether with a high purity from 2-methoxyethanol and acetylene.

### Means of Solving the Problem

The problem according to the present invention has been solved by the following means <1> to <6>.
<1> A method for producing (2-methoxyethyl) vinyl ether using 2-methoxyethanol as a raw material alcohol, the method including the following steps A1, A2, and A3:
   (Step A1) a vinyl ether synthesis step of causing 2-methoxyethanol to react with acetylene to obtain a mixture containing unreacted 2-methoxyethanol and (2-methoxyethyl) vinyl ether;
   (Step A2) an acetalization step of causing a reaction of the unreacted 2-methoxyethanol and the (2-methoxyethyl) vinyl ether in the mixture in the presence of an acid catalyst to be converted into acetaldehyde bis(2-methoxyethyl)acetal;
   (Step A3) a distillation step of removing the acetaldehyde bis(2-methoxyethyl)acetal by distillation from an acetal-containing mixture obtained in step A2.
<2> A method for producing (2-methoxyethyl) vinyl ether using 2-methoxyethanol as a raw material alcohol, the method including the following steps A1, A2, A2-2, and A3.
   (Step A1) a vinyl ether synthesis step of causing 2-methoxyethanol to react with acetylene to obtain a mixture containing unreacted 2-methoxyethanol and (2-methoxyethyl) vinyl ether;
   (Step A2) an acetalization step of causing a reaction of the unreacted 2-methoxyethanol and the (2-methoxyethyl) vinyl ether in the mixture in the presence of an acid catalyst to be converted into acetaldehyde bis(2-methoxyethyl)acetal;
   (Step A2-2) a step of performing a treatment selected from the group consisting of neutralization of the acid catalyst and removal of the acid catalyst; and
   (Step A3) a distillation step of removing the acetaldehyde bis(2-methoxyethyl)acetal by distillation from an acetal-containing mixture obtained in step A2-2.
<3> The production method according to <2>, wherein step A2-2 is a step of performing a neutralization treatment of the acid catalyst using a basic compound.
<4> The production method according to any one of <1> to <3>, wherein step A1 is performed in the presence of an alkali metal alcoholate catalyst.
<5> The production method according to any one of <1> to <4>, wherein a distillation pressure in step A3 is 40 kPaA to atmospheric pressure.
<6> A method for purifying (2-methoxyethyl) vinyl ether from a mixture containing 2-methoxyethanol and (2-methoxyethyl) vinyl ether, the method including the following steps B1 and B2:
   (Step B1) an acetalization step of causing a reaction of 2-methoxyethanol and (2-methoxyethyl) vinyl ether in the mixture in the presence of an acid catalyst to be converted into acetaldehyde bis(2-methoxyethyl)acetal;
   (Step B2) a distillation step of removing the acetaldehyde bis(2-methoxyethyl)acetal by distillation from an acetal-containing mixture obtained in step B1.

### Effects of the Invention

The production method of the present invention can produce (2-methoxyethyl) vinyl ether with a high purity from 2-methoxyethanol and acetylene.

Further, the purification method of the present invention can purify (2-methoxyethyl) vinyl ether with a high purity from a mixture containing 2-methoxyethanol and (2-methoxyethyl) vinyl ether.

### Detailed Description of the Invention

### [Method for producing (2-methoxyethyl) vinyl ether]

The production method of the present invention is a method for producing (2-methoxyethyl) vinyl ether using 2-methoxyethanol as a raw material alcohol, and includes the steps A1, A2, and A3. Specifically, after step A1 (a vinyl ether synthesis step) is performed, step A1-2 (a catalyst removal step) is performed, if necessary, on a mixture containing an unreacted raw material alcohol and (2-methoxyethyl) vinyl ether obtained in the step (a mixture further containing a catalyst in a case where a vinyl ether synthesis is performed in the presence of a catalyst), and then step A3 (a distillation step) is performed after step A2 (an acetalization step).

### (Step A1 Vinyl ether synthesis step)

Step A1 is a vinyl ether synthesis step of causing 2-methoxyethanol (boiling point: 124°C) to react with acetylene to obtain a mixture containing an unreacted raw material alcohol and (2-methoxyethyl) vinyl ether (boiling point: 109°C). (2-methoxyethyl) vinyl ether is a low boiling point vinyl ether, and according to the production method of the present invention, such (2-methoxyethyl) vinyl ether with a high purity, being a low boiling point vinyl ether, can be produced.

Further, an oxyethylene group-containing vinyl ether is generally hydrolyzed in the presence of an acid and decomposed into an alcohol and aldehyde (for example, JP 2019-44050 A). However, as recited in examples to be described later, (2-methoxyethyl) vinyl ether with a high purity can be efficiently produced despite being an oxyethylene group-containing vinyl ether.

Step A1 is preferably performed in the presence of a catalyst. As the catalyst, an alkali metal alcoholate catalyst is preferable from the viewpoint of reaction efficiency.

The alkali metal alcoholate catalyst is an alkali metal alcoholate of an alkali metal hydroxide and 2-methoxyethanol, and is preferably dissolved in 2-methoxyethanol from a viewpoint of handleability. Specific examples of the alkali metal hydroxide include sodium hydroxide, potassium hydroxide, rubidium hydroxide, and cesium hydroxide, and one of these may be used alone, or two or more kinds thereof may be used in combination.

Step A1 may be performed in the presence of an organic solvent or in the absence of an organic solvent, but is preferably performed without a solvent.

As the organic solvent, for example, an aprotic polar solvent that is mixed with 2-methoxyethanol and dissolves an alkali metal alcoholate catalyst is preferable. Examples thereof include amide solvents such as dimethylacetamide, 2-pyrrolidone, N-methyl-2-pyrrolidone, and 1,3-dimethyl-2-imidazolidinone; sulfur-containing compound solvents such as sulfolane and dimethyl sulfoxide; and glycol dialkyl ether solvents such as diethylene glycol dimethyl ether, diethylene glycol diethyl ether, triethylene glycol dimethyl ether, triethylene glycol diethyl ether, tetraethylene glycol dimethyl ether, and tetraethylene glycol diethyl ether. One of these solvents may be used alone, or two or more kinds thereof may be used in combination.

A reaction temperature in step A1 is usually in a range of 0 to 180°C, and more preferably in a range of 90 to 140°C from a viewpoint of reaction rate and side reaction restraint. The reaction rate increases as a reaction pressure increases, but it is preferable to set the reaction pressure to 0.3 MPa or less in order to prevent decomposition explosion of acetylene. Further, reaction time of step A1 is usually about 10 minutes to 200 hours.

### (Step A1-2 Catalyst removal step)

In the production method of the present invention, when the vinyl ether synthesis is performed in the presence of a catalyst, the catalyst, for example, may be removed from a reaction mixture obtained in step A1 prior to step A2.

Removal of the catalyst, for example, can be performed by a known method such as solid-liquid separation (in a case of a solid catalyst or a supported catalyst) such as solvent extraction, distillation, and filtration. Among these methods, a method by distillation is preferable in that the catalyst can be easily separated and the raw material alcohol can be reduced in advance. Further, in a case of the method by distillation, the organic solvent at the time of performing step A1 using an organic solvent can also be removed. Further, when the catalyst is removed by a method other than distillation, further distillation may be performed to reduce the raw material alcohol in the reaction mixture.

A distillation column used for removal of the catalyst, for example, or distillation for concentration after removal of the catalyst (hereinafter, also referred to as "preliminary distillation") may be any one of a packed column, a plate column, a bubble cap column, for example, and number of plates of the distillation column is, for example, 1 to 100, and preferably 5 to 50 in term of theoretical plates.

The preliminary distillation may be performed under any condition of normal pressure, increased pressure, and reduced pressure, and is preferably performed under normal pressure or reduced pressure. Specifically, the pressure is usually 0.7 to 13.3 kPa, and preferably 1.3 to 6.7 kPa. Further, a distillation method may be any of a batch method, a semi-batch method, or a continuous method.

### (Step A2 Acetalization step)

Step A2 is an acetalization step of causing a reaction of the unreacted raw material alcohol and (2-methoxyethyl) vinyl ether in the mixture obtained in step A1 or step A1-2 in the presence of an acid catalyst to convert them into acetaldehyde bis(2-methoxyethyl)acetal.

A combination of 2-methoxyethanol as a raw material alcohol and (2-methoxyethyl) vinyl ether forms an azeotropic mixture, but by performing step A2, the unreacted raw material alcohol is subjected to acetalization, and a purity of the (2-methoxyethyl) vinyl ether can be easily increased by distillation operation, despite the fact that a raw material alcohol and a target vinyl ether form an azeotropic mixture in this manner.

Examples of the acid catalyst used in step A2 include inorganic acids such as sulfuric acid, nitric acid, hydrochloric acid, and phosphoric acid; organic acids such as carboxylic acid and organic sulfonic acid; and solid acid catalysts such as acidic zeolite, heteropoly acid, and a strong acid ion□exchange resin. One of these solvents may be used alone, or two or more kinds thereof may be used in combination.

Among them, from the viewpoint of side reaction restraint (particularly, restraint of polymerization reaction of vinyl ether), phosphoric acid, organic sulfonic acid, and a sulfonic acid type strong acid ion-exchange resin (a strong acid ion-exchange resin having a sulfonic acid group in the molecule) are preferable, and organic sulfonic acid is more preferable. When the organic sulfonic acid is used, (2-methoxyethyl) vinyl ether is less likely to be hydrolyzed, so that (2-methoxyethyl) vinyl ether can be further purified.

Examples of the organic sulfonic acid include aromatic sulfonic acids such as p-toluene sulfonic acid, o-toluene sulfonic acid, benzenesulfonic acid, p-xylene-2-sulfonic acid, dodecylbenzene sulfonic acid, 1-naphthalene sulfonic acid, 2-naphthalene sulfonic acid, dinonylnaphthalene sulfonic acid, and dinonylnaphthalene disulfonic acid; aliphatic sulfonic acids such as methane sulfonic acid, ethane sulfonic acid, and trifluoromethanesulfonic acid; and aromatic sulfonates such as pyridinium p-toluene sulfonate and quinolinium p-toluene sulfonate.

Examples of the strong acid ion-exchange resin include a sulfonic acid type strong acid ion-exchange resin and a mixture of a sulfonic acid type strong acid ion-exchange resin and an amine type weakly basic ion-exchange resin. Examples of commercially available products of the sulfonic acid type strong acid ion-exchange resin include Amberlyst 15DRY manufactured by Organo Corporation, and examples of commercially available products of a mixture of the sulfonic acid type strong acid ion-exchange resin and the amine type weakly basic ion-exchange resin include Amberlyst MSPS 2-1 · DRY manufactured by Organo Corporation.

From the viewpoint of purity of (2-methoxyethyl) vinyl ether, amount of the acid catalyst used is usually 0.00001 to 5 parts by mass, preferably 0.0001 to 1 part by mass, more preferably 0.001 to 0.1 part by mass, still more preferably 0.001 to 0.01 part by mass, and still more preferably 0.005 to 0.01 part by mass, based on 100 parts by mass of (2-methoxyethyl) vinyl ether.

A method for adding an acid catalyst to the mixture obtained in step A1 or step A1-2 may be appropriately selected according to a type of the acid catalyst. For example, in a case of an inorganic acid or an organic acid, the acid may be added to the mixture obtained in step A1 or step A1-2 as it is or after being dissolved in an appropriate solvent (which is preferably 2-methoxyethanol). Further, in a case of solid acid catalyst, the solid acid catalyst may be directly added to the mixture obtained in step A1 or step A1-2, or the solid acid catalyst may be packed in a column container, for example, and the mixture obtained in step A1 or step A1-2 may be passed through the column container, for example.

The reaction temperature in step A2 is preferably in a range of 0 to 80°C, more preferably in a range of 10 to 60°C, from the viewpoint of reaction rate and side reaction restraint.

The reaction time of step A2 is usually about 10 minutes to 48 hours.

The acetal obtained in step A2 is acetaldehyde bis(2-methoxyethyl)acetal (boiling point: 197°C).

Further, the production method of the present invention preferably includes a step of performing a treatment selected from the group consisting of neutralization of the acid catalyst and removal of the acid catalyst (step A2-2) after step A2 and prior to step A3, in order to suppress production of a heavy material in step A3.

Further, when an inorganic acid or an organic acid is used as the acid catalyst, neutralization of the acid catalyst is preferable as step A2-2. The neutralization of the acid catalyst may be performed using a basic compound. Examples of the basic compound include alkali metal compounds such as alkali metal hydroxides (for example, sodium hydroxide and potassium hydroxide), alkali metal carbonates, and alkali metal hydrogen carbonates; and a basic ion-exchange resin. Further, examples of the alkali metal include sodium and potassium.

From the viewpoint of the purity of (2-methoxyethyl) vinyl ether, amount of the basic compound used is usually 1 to 1000 molar equivalents, preferably 1 to 100 molar equivalents, more preferably 5 to 100 molar equivalents, and particularly preferably 8 to 50 molar equivalents, relative to the acid catalyst used in step A2. When the amount of the basic compound used is 8 molar equivalents or more, (2-methoxyethyl) vinyl ether is obtained particularly efficiently.

When an alkali metal compound is used as the basic compound, the alkali metal compound may be added to an acetal-containing mixture obtained in step A2 as it is or after being dissolved in an appropriate solvent (which is preferably 2-methoxyethanol). Further, when the basic ion-exchange resin is used, the basic ion-exchange resin may be directly added to the acetal-containing mixture obtained in step A2, or the basic ion-exchange resin may be packed in a column container, and the acetal-containing mixture obtained in step A2 may be passed through the column container. Further, when a solid or a precipitate is present in a liquid after neutralization, the solid or the precipitate may be subjected to solid-liquid separation by filtration or centrifugation as necessary.

On the other hand, when, for example, a solid acid catalyst is used as the acid catalyst, removal of the acid catalyst is preferable as step A2-2. Examples of a removal operation include solid-liquid separation operations such as filtration and centrifugation. Note that, when the solid acid catalyst is packed in a column container and used, a separation operation is unnecessary.

### (Step A3 Distillation step)

Step A3 is a distillation step of removing the acetaldehyde bis(2-methoxyethyl)acetal by distillation from the acetal-containing mixture obtained in step A2 (step A2-2 when step A2-2 is performed).

A distillation apparatus and distillation method to be used in step A3 are not particularly limited, and number of distillation plates may be provided even in a simple distillation. The distillation method may be any of a batch method, a semi-batch method, and a continuous method. Further, when a distillation column is used, the distillation column may be, for example, any of a packed column, a plate column, and a bubble cap column. The number of plates of the distillation column is preferably 1 to 30 plates, more preferably 5 to 15 plates in terms of theoretical plates. Further, a reflux ratio is preferably in a range of 1 to 15.

Further, an overhead temperature of the distillation column is preferably 50 to 120°C and more preferably 70 to 110°C, and a bottom temperature of the distillation column is preferably 50 to 210°C and more preferably 70 to 200°C.

A distillation pressure in step A3 is preferably 20 to 120 kPaA (where A represents an absolute pressure), more preferably 40 kPaA to atmospheric pressure from the viewpoint of the purity of (2-methoxyethyl) vinyl ether.

When a distillation column is used, the target (2-methoxyethyl) vinyl ether with a high purity is obtained from the overhead of the distillation column, and a bottom liquid rich in acetaldehyde bis(2-methoxyethyl)acetal is recovered from the bottom of the column. The acetaldehyde bis(2-methoxyethyl)acetal contained in the bottom liquid can be recovered, converted into vinyl ether and a raw material alcohol, and recycled as a raw material for (2-methoxyethyl) vinyl ether synthesis.

### (Step A4 Acetal decomposition step)

As a method for converting acetaldehyde bis(2-methoxyethyl)acetal into a raw material alcohol and vinyl ether, a known method may be appropriately used. Specific examples thereof include a method of thermally decomposing in a gas phase in the presence of a silica/alumina-based catalyst carrying an alkali or alkaline earth metal (for example, Khim. Prom.48(9)657-660(1972), JP 48-78109 A, JP 62-87247 A, for example), a method of decomposing in a gas phase using magnesium oxide as a catalyst (JP H8-268945 A), a method of decomposing in the presence of a catalyst containing a noble metal (for example, Ann.,601 81-84,1956, DE 1957680 A1, JP 48-76803 A, for example), and a method of decomposing using an acid catalyst (for example, J.Org.Chem.,38,2910,1973, Helv.Chim.Acta,1158 (1967), Bull.Chem.Soc.Jpn.3089(1976), JP H8-277237 A, for example).

### [Method for purifying (2-methoxyethyl) vinyl ether]

The purification method of the present invention is a method for purifying (2-methoxyethyl) vinyl ether from a mixture containing 2-methoxyethanol and (2-methoxyethyl) vinyl ether, and includes the following steps B1 and B2.
(Step B1) an acetalization step of causing a reaction of 2-methoxyethanol and (2-methoxyethyl) vinyl ether in the mixture in the presence of an acid catalyst to be converted into acetaldehyde bis(2-methoxyethyl)acetal;
(Step B2) a distillation step of removing the acetaldehyde bis(2-methoxyethyl)acetal by distillation from an acetal-containing mixture obtained in step B1.

Step B1 may be performed in the same manner as step A2 in the production method of the present invention, and step B2 may be performed in the same manner as step A3 in the production method of the present invention.

### Examples

Hereinafter, the present invention will be described in detail with reference to examples. But, the present invention is not limited to these examples.

### [Example 1 Production of (2-methoxyethyl) vinyl ether (MOVE)]

### (Vinyl ether synthesis step and catalyst removal step)

A stainless steel autoclave with a volume of 10 L was used as a reaction vessel, and a stainless steel packed type continuous distillation column (packing: Sumitomo Heavy Industries, Ltd., Sumitomo/Sulzer Laboratory packing) with an inner diameter of 60 mmϕ, 12 theoretical plates (with seven supply plates) and a pot volume of 4 L was used as a continuous distillation column.

An autoclave was filled with 6.2 kg of 2-methoxyethanol and 1.4 kg of potassium hydroxide. An alcoholate catalyst (potassium-2-methoxyethane-1-olate) was prepared for 9 hours under autoclave conditions: 125°C, and 0.03 MPaG, and continuous distillation column conditions: an overhead temperature of 70°C, a bottom temperature of 140°C, 20 kPa, a reflux ratio of 5, and a circulation flow rate between the autoclave and the distillation column of 15 kg/hr. The amount of distillate from the overhead of the continuous distillation column during this period was 0.96 kg, containing 0.38 kg of water.

Next, the autoclave conditions were changed to 120°C and 0.04 MPaG, the continuous distillation column conditions were changed to conditions of a reflux ratio of 5, an overhead pressure of 40 kPa, an overhead temperature of 80°C, and a bottom temperature of 140°C, and acetylene and 2-methoxyethanol were continuously supplied at rates of 94 g/hr and 220 g/hr, respectively.

In this way, a crude vinyl ether was continuously synthesized, and the crude vinyl ether was obtained at a flow rate of 291 g/hr from the overhead of the continuous distillation column. Here, composition of the crude vinyl ether was 90.6 mass% of (2-methoxyethyl) vinyl ether and 9.4 mass% of 2-methoxyethanol.

### (Acetalization reaction step and distillation purification step)

A crude MOVE obtained in the above step was weighed into a 2000 mL three-necked flask equipped with a stirrer tip, 0.6 g of a 2-methoxyethanol solution of p-toluenesulfonic acid (p-toluenesulfonic acid concentration: 9.6 mass%, p-toluenesulfonic acid at 59 ppm relative to the crude MOVE) was added thereto, and the mixture was stirred in a water bath set at 25°C for 60 minutes. The amount of 2-methoxyethanol in the solution after the reaction was 0.1 mass% or less, and MOVE and acetaldehyde bis(2-methoxyethyl)acetal were contained in the amounts of 83 mass% and 17 mass%, respectively. After neutralization by adding 1.45 g of a 2-methoxyethanol solution of potassium hydroxide (potassium hydroxide concentration: 10 mass%, potassium hydroxide at 170 ppm relative to the reaction solution, 8.8 molar equivalents relative to p-toluene sulfonic acid) to the reaction solution, distillation was performed using a packed column having 10 theoretical plates (internal pressure: 60 kPaA, column bottom setting temperature: 100 to 140°C, refrigerant setting temperature: 5°C) to obtain a high-purity MOVE having a purity of 99 mass% or more (recovery rate based on MOVE: 79.8 mass%).

## Claims

1. A method for producing (2-methoxyethyl) vinyl ether using 2-methoxyethanol as a raw material alcohol, the method comprising the following steps A1, A2 and A3:
(Step A1) a vinyl ether synthesis step of causing 2-methoxyethanol to react with acetylene to obtain a mixture containing unreacted 2-methoxyethanol and (2-methoxyethyl) vinyl ether;
(Step A2) an acetalization step of causing a reaction of the unreacted 2-methoxyethanol and the (2-methoxyethyl) vinyl ether in the mixture in the presence of an acid catalyst to be converted into acetaldehyde bis(2-methoxyethyl)acetal; and
(Step A3) a distillation step of removing the acetaldehyde bis(2-methoxyethyl)acetal by distillation from an acetal-containing mixture obtained in step A2.

2. A method for producing (2-methoxyethyl) vinyl ether using 2-methoxyethanol as a raw material alcohol, the method comprising the following steps A1, A2, A2-2 and A3:
(Step A1) a vinyl ether synthesis step of causing 2-methoxyethanol to react with acetylene to obtain a mixture containing unreacted 2-methoxyethanol and (2-methoxyethyl) vinyl ether;
(Step A2) an acetalization step of causing a reaction of the unreacted 2-methoxyethanol and the (2-methoxyethyl) vinyl ether in the mixture in the presence of an acid catalyst to be converted into acetaldehyde bis(2-methoxyethyl)acetal;
(Step A2-2) a step of performing a treatment selected from the group consisting of neutralization of the acid catalyst and removal of the acid catalyst; and
(Step A3) a distillation step of removing the acetaldehyde bis(2-methoxyethyl)acetal by distillation from an acetal-containing mixture obtained in Step A2-2.

3. The production method according to Claim 2, wherein step A2-2 is a step of performing a neutralization treatment of the acid catalyst using a basic compound.

4. The production method according to any one of Claims 1 to 3, wherein step A1 is performed in the presence of an alkali metal alcoholate catalyst.

5. The production method according to any one of Claims 1 to 4, wherein a distillation pressure in step A3 is 40 kPaA to atmospheric pressure.

6. A method for purifying (2-methoxyethyl) vinyl ether from a mixture containing 2-methoxyethanol and (2-methoxyethyl) vinyl ether, the method comprising the following steps B1 and B2:
(Step B1) an acetalization step of causing a reaction of 2-methoxyethanol and (2-methoxyethyl) vinyl ether in the mixture in the presence of an acid catalyst to be converted into acetaldehyde bis(2-methoxyethyl)acetal; and
(Step B2) a distillation step of removing the acetaldehyde bis(2-methoxyethyl)acetal by distillation from an acetal-containing mixture obtained in step B1.
